# EUROPEAN PATENT APPLICATION

(11) **EP 4 834 245 A1**
(43) Date of publication of application: **04.11.2026**
(21) Application number: 25747618.4
(22) Date of filing: 21.01.2025
(51) Int. Cl.: A61B 17/12

(54) **OCCLUDER AND OCCLUSION SYSTEM**

(30) Priority: 02.02.2024 CN 202410153683
(71) Applicant: Shanghai Microport Cardioadvent Co., Ltd., Shanghai 201203 (CN)
(72) Inventor: ZHU, Zexun, Shanghai 201203 (CN); YAO, Yao, Shanghai 201203 (CN); REN, Kaibing, Shanghai 201203 (CN); LI, Junfei, Shanghai 201203 (CN)
(74) Representative: Marks & Clerk LLP
(86) International application number: PCT/CN2025/073518
(87) International publication number: WO 2025/162046

(57) **Abstract**

An occluder (1) and an occlusion system, the occluder (1) includes a mesh body (10) and a plurality of cushioning elements (20), and comprises open and closed ends along an axis, and comprises a collapsed configuration and an expanded configuration. When the occluder (1) is in the expanded configuration, the mesh body (10) comprises a ring shape about the axis, and the multiple cushioning elements (20) are circumferentially arranged at the open end about the axis. A perpendicular bisector plane of each cushioning element (20) passing through the axis defines a reference plane (30). The cushioning element (20) is asymmetrical about its own reference plane (30). Such asymmetry arrangement of each cushioning element (20) about its reference plane (30) can effectively reduce entanglement or interdigitating of the cushioning elements (20), thereby enhancing the stability of the occluder without compromising its performance during use. In addition, the presence of the cushioning elements (20) helps reduce damage to tissue, contributing to increased safety thereof.

## Description

### TECHNICAL FIELD

The present invention relates to the field of medical devices, and more particularly to an occluder and an occlusion system.

### BACKGROUND

Studies have shown that left atrial appendage (LAA) occlusion allows for effective prevention of an atrial fibrillation (AF)-induced stroke.

LAA occlusion can be accomplished using an occluder. There has been disclosed a cage-like internal occluder, which is designed to be deployed in an LAA and anchored therein with hooks of the occluder.

However, this cage-like internal occluder exhibits a high density at its distal end, which makes the occluder prone to interdigitating or entanglement of adjacent structures thereof during its unloading from a sheath after it has been loaded. When this takes place, complete expansion of a stent of the occluder may become impossible. Consequently, the actual size of the expanded stent may not be compatible with the size of a cavity to be filled, as desired, leading to malapposition and hence instability of the stent. The high density at the distal end also creates difficulties in crimping and loading of the occluder into the sheath, as well as in retrieval and delivery thereof. Further, such a distal end of the cage-like internal occluder tends to cause damage to tissue.

### SUMMARY OF THE INVENTION

It is an object of the present invention to address the problem of easy interdigitating or entanglement associated with the existing occluders by presenting a novel occluder and occlusion system.

The above object is accomplished according to the present invention by providing an occluder, which comprises a mesh body and a plurality of cushioning elements,
wherein the occluder comprises an open end and a closed end along an axis, and comprises a collapsed configuration and an expanded configuration, wherein when the occluder is in the expanded configuration, the mesh body comprises a ring shape about the axis, and wherein the plurality of cushioning elements are circumferentially arranged at the open end about the axis,
wherein a perpendicular bisector plane of the cushioning element passing through the axis defines a reference plane, and wherein the cushioning element is asymmetrical about the reference plane.

Optionally, when the occluder is in the expanded configuration, each cushioning element may be inwardly inclined towards the axis along a direction away from the mesh body.

Optionally, when the occluder is in the expanded configuration, each cushioning element comprises a planar shape perpendicular to the reference plane, or a comprises a three-dimensional shape.

Optionally, the mesh body may comprise a plurality of first wave struts arranged circumferentially arranged about the axis,
wherein each cushioning element comprises two second wave struts and a curved strut, wherein the first ends of the two second wave struts joined to two circumferentially adjacent first wave struts, thereby forming two first nodes, and wherein second ends of the two second wave struts are joined to each other to form a second node,
wherein a first end of the curved strut is joined to the second node, and wherein a second end of the curved strut is joined to the mesh body.

Optionally, the second end of the curved strut is affixed at the first node, wherein the second ends of the curved struts of circumferentially adjacent cushioning elements are affixed at different first nodes.

Optionally, the first wave strut is inclined relative to the axis, wherein circumferentially adjacent first wave struts comprise opposite inclination directions, wherein the plurality of first wave struts are joined sequentially to form a zigzag-shaped ring, and wherein ends of circumferentially adjacent first wave struts towards the cushioning elements are joined together and are further joined to the second wave strut to from the first node.

Optionally, the first wave strut is inclined relative to the axis, wherein circumferentially adjacent first wave struts comprise opposite inclination directions, wherein the plurality of first wave struts are joined sequentially to form a zigzag-shaped ring; ends of circumferentially adjacent first wave struts towards the cushioning elements are joined together and are further joined to the second wave strut to form the first node, and wherein ends of circumferentially adjacent first wave struts away from the cushioning element are joined together to form a third node; and
wherein the second end of the curved strut is affixed at the third node, and wherein the second ends of the curved struts of circumferentially adjacent cushioning elements are affixed at different third nodes.

Optionally, the second end of the curved strut of the cushioning element is joined to the third node formed by the first wave struts that are joined to the two second wave struts of the same cushioning element.

Optionally, in each cushioning element, one end of at least one of the second wave struts away from the first node comprises a curved section, and/or wherein the curved strut is curved inwardly or outwardly with respect to the mesh body.

The above object is also accomplished according to the present invention by providing an occlusion system, which comprises an occluder as defined above and a delivery device configured to load the occluder in the collapsed configuration and to release the occluder.

In summary, the present invention provides an occluder and an occlusion system. The occluder includes a mesh body and a plurality of cushioning elements, and comprises open and closed ends along an axis. The occluder comprises a collapsed configuration and an expanded configuration. When the occluder is in the expanded configuration, the mesh body comprises a ring shape about the axis, and the multiple cushioning elements are circumferentially arranged at the open ends about the axis. A perpendicular bisector plane of each cushioning element passing through the axis defines a reference plane. Each cushioning element is asymmetrical about its reference plane.

With this arrangement, the asymmetry arrangement of each cushioning element about its reference plane can effectively reduce entanglement or interdigitating of the cushioning elements, thereby enhancing the stability of the occluder without compromising its performance during use. In addition, the presence of the cushioning elements helps reduce damage to tissue, contributing to increased safety thereof.

### BRIEF DESCRIPTION OF THE DRAWINGS

Those of ordinary skill in the art will understand that the following drawings are presented to enable a better understanding of the present invention and not intended to limit the scope thereof in any sense, in which:
Fig. 1 shows a perspective view of an occluder, in accordance with an embodiment of the present invention;
Fig. 2 shows a schematic cross-sectional view of the occluder, in accordance with an embodiment of the present invention, taken along an axis thereof;
Fig. 3 shows a perspective view of a first example of cushioning elements and a mesh body, in accordance with an embodiment of the present invention;
Fig. 4 shows a schematic side view of the first example of the cushioning elements and mesh body, in accordance with an embodiment of the present invention;
Fig. 5 shows a schematic bottom view of the first example of the cushioning elements and mesh body, in accordance with an embodiment of the present invention;
Fig. 6 is a schematic illustration of an occlusion system, in accordance with an embodiment of the present invention;
Fig. 7 schematically illustrates a relative relationship between the mesh body and an LAA in the case of the cushioning elements being omitted;
Fig. 8 schematically illustrates a relative relationship between the cushioning elements and the LAA, in accordance with an embodiment of the present invention;
Fig. 9a schematically illustrates two curved struts being joined to a single first node;
Fig. 9b schematically illustrates a single curved strut being joined to a single first node;
Fig. 10 is a schematic diagram showing a second example of the cushioning elements and mesh body, in accordance with an embodiment of the present invention, in which the mesh body includes a single row of struts;
Fig. 11 is a schematic diagram showing the second example of the cushioning elements and mesh body, in accordance with an embodiment of the present invention, in which the mesh body includes two rows of struts;
Fig. 12 shows a perspective view of a third example of the cushioning elements and mesh body, in accordance with an embodiment of the present invention;
Fig. 13 shows a side view of the third example of the cushioning elements and mesh body, in accordance with an embodiment of the present invention;
Fig. 14 shows a top view of the third example of the cushioning elements and mesh body, in accordance with an embodiment of the present invention;
Fig. 15 schematically illustrates a relative relationship between a cushioning element, the mesh body and an LAA in the third example, in accordance with an embodiment of the present invention; and
Fig. 16 shows a side view of a fourth example of the cushioning elements and mesh body, in accordance with an embodiment of the present invention.

### List of Reference Numerals

1 occluder; 10 mesh body; 101 first node; 103 third node; 11 first wave strut; 13 third wave strut; 14 fourth wave strut; 20 cushioning element; 202 second node; 21 second wave strut; 211 curved section; 22 curved strut; 30 reference plane; 40 converging member; 41 converging strut; 42 attachment member; 50 graft; 60 delivery device; 62 push member; 63 sheath; 70 LAA.

### DETAILED DESCRIPTION

Objects, advantages and features of the present invention will become more apparent upon reading the following more detailed description with reference to the accompanying drawings, which illustrate particular embodiments thereof. Note that the figures are heavily simplified and not necessarily drawn to scale. They are presented only to assist in facilitating clear illustration of the embodiments disclosed herein. In addition, the illustrated features are usually partial representations of their real-world counterparts. In particular, as the figures tend to highlight different features, different scales may be used accordingly.

As used herein, the singular forms "a", "an" and "the" include plural referents, and the term "or" is generally employed in the sense of "and/or", "a number of" is generally employed in the sense of "at least one" and "at least two" is generally employed in the sense of "two or more". In addition, the terms such as "first," "second," "third" and the like are intended only for illustration, and are not to be construed as denoting or implying relative importance, or as implicitly indicating any particular number of the referenced items. Accordingly, defining an item with "first," "second," or "third" is an explicit or implicit indication of the presence of one or at least two such items. The terms "one end" and "other end", as well as "proximal end" and "distal end", may be used herein to generally refer to opposite end portions including the respective endpoints, rather than only to the endpoints. Further, as used herein, the terms "mounting", "coupling", "connecting", "disposing" and any variants thereof should be interpreted in a broad sense. When an element is referred to as being "disposed" on another element, this is generally intended to only mean that there is a connection, coupling, engagement or transmission relationship between the two elements, which may be either direct or indirect with one or more intervening elements, and should not be interpreted as indicating or implying a particular spatial position relationship between them. That is, the element may be located inside, outside, above, under, beside, or at any other location relative to the other element, unless the context clearly dictates otherwise. Those of ordinary skill in the art can understand the specific meanings of the above-mentioned terms herein, depending on their context. Furthermore, the directional terms such as "above", "below", "upper", "lower", "upward", "downward", "left", "right", and the like are used in relation to the illustrative embodiments as they are depicted in the figures, the upward or upper direction being toward the top of the corresponding figure and the downward or lower direction being toward the bottom of the corresponding figure.

It is an object of the present invention to address the problem of easy interdigitating or entanglement associated with the existing occluders by presenting a novel occluder and occlusion system. The invention is described below with reference to the appended figures.

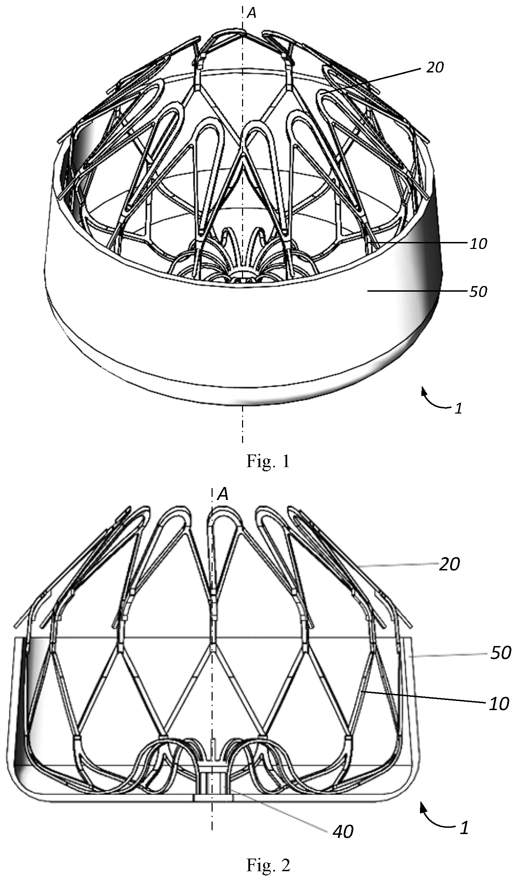

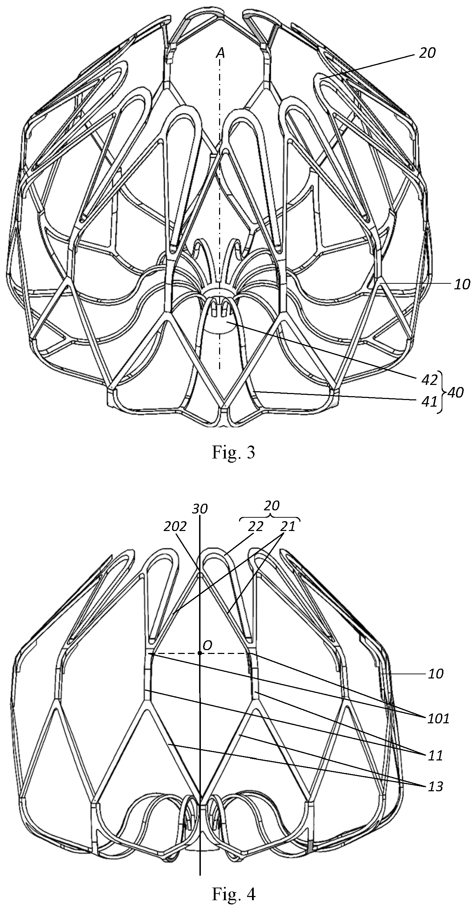

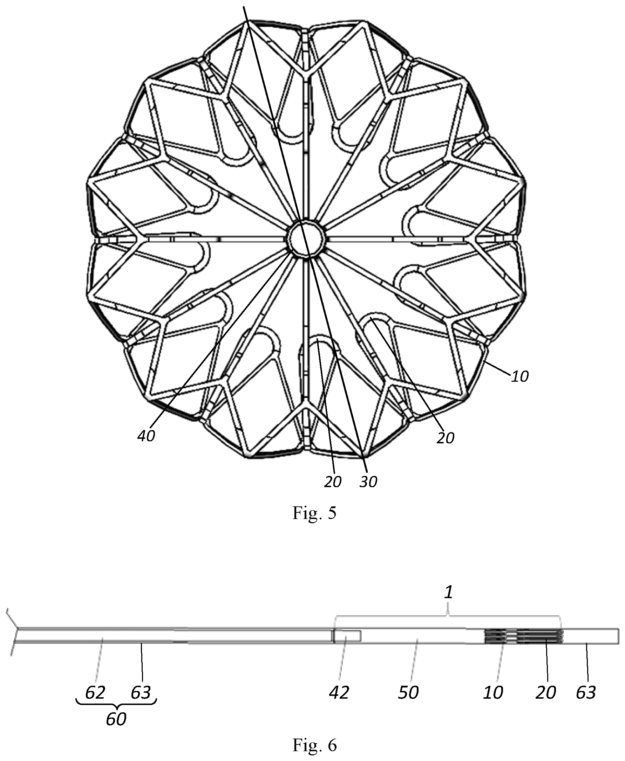

Referring to Figs. 1 to 5, embodiments disclosed herein provide an occluder 1 essentially used to occlude a left atrial appendage (LAA). The occluder 1 includes a mesh body 10 and a plurality of cushioning elements 20. The occluder 1 has an open end (the upper end as viewed in the orientation of Figs. 1 and 2, configured for deployment within a cavity of the LAA) and a closed end (the lower end as viewed in the orientation of Figs. 1 and 2, configured for closure of the orifice of the LAA), along an axis A. The occluder 1 comprises a collapsed configuration and an expanded configuration. In the expanded configuration of the occluder 1, the mesh body 10 comprises a ring shape about the axis A, and the plurality of cushioning elements 20 are arranged circumferentially about the axis A at the open end. A perpendicular bisector plane of the cushioning element 20 passing through the axis A defines a reference plane 30 (see Figs. 4 and 5). Each cushioning element 20 is asymmetrical about the reference plane 30 thereof. It should be noted that the reference plane 30 is an imaginary plane, rather than a physical structure present in the occluder 1. Further, in order to more clearly illustrate the reference plane 30, only portions facing the viewer (circumferential halves), rather than the entirety, of the cushioning element 20 and mesh body 10 are shown in Fig. 4.

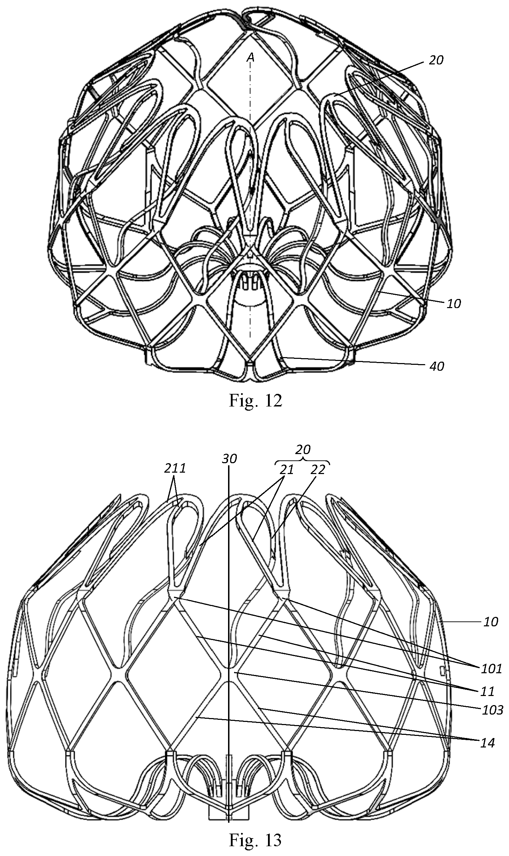

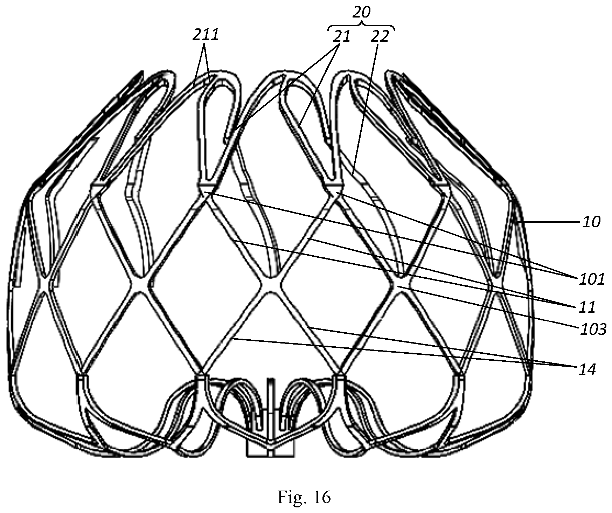

It should be noted that the joint of each cushioning element 20 and the mesh body 10 comprises a width along the circumference of the mesh body 10. For example, each cushioning element 20 may be joined to the mesh body 10 at two first nodes 101 (as shown in Fig. 4 and described below), and the length of a line segment connecting the two first nodes 101 may represent a width of the cushioning element 20 along the circumference of the mesh body 10. Moreover, the perpendicular bisector plane of the cushioning element 20 may be defined as a plane passing through the midpoint O of the line segment that connects the two first nodes 101 and the axis A. In some embodiments, each cushioning element 20 is joined to the mesh body 10 at three different nodes, namely, two first nodes 101 and a third node 103 (as shown in Figs. 13 and 16). In these embodiments, the perpendicular bisector plane of the cushioning element 20 is defined as a plane passing through a centroid defined by the three nodes of the cushioning elements 20 (the center of a shape formed by connecting the three nodes) and the axis A. In Fig. 4, the reference plane 30 runs perpendicular to the plane of the drawing. In the illustrated embodiments, each cushioning element 20 is asymmetrical about its reference plane 30, in the occluder 1.

Here, it will be understood that, by "each cushioning element 20 is asymmetrical about its reference plane 30", it is intended to mean that each cushioning element 20 itself is an asymmetrical structure. Moreover, since the plurality of cushioning elements 20 are arranged circumferentially about the axis A, an assembly of all the cushioning elements 20 is also asymmetrical about the reference plane 30 of each cushioning element 20. That is, this assembly is also an asymmetrical structure. Studies have shown that such an asymmetrical structure of the cushioning elements 20 can mitigate or eliminate entanglement or interdigitating of the plurality of cushioning elements 20, while the occluder 1 is being pushed out of a sheath 63, or during its unloading from the sheath 63 after it has been loaded, thereby enhancing the stability of the occluder without compromising its performance during use.

Therefore, with this arrangement, the asymmetry of each cushioning element 20 about its reference plane 30 can effectively reduce entanglement or interdigitating of the cushioning elements 20, thereby enhancing the stability of the occluder without compromising its performance during use. In addition, the presence of the cushioning elements 20 helps reduce damage caused by to tissue, contributing to increased safety thereof.

With additional reference to Fig. 6, as would be appreciated, when the occluder 1 is in the collapsed configuration, the mesh body 10 is still arranged about the axis A, and the mesh frame main body 10 is relatively compactly arranged and does not necessarily comprise a ring shape. The present invention is not limited to any particular shape of the occluder 1 in the collapsed configuration, and a person of skill in the art can properly configure the shape, as required by the application.

For ease of description, the two ends along the axis A can also be referred to herein as a proximal end (the left-hand end as viewed in the orientation of Fig. 6) and a distal end (the right-hand end as viewed in the orientation of Fig. 6). The distal end of the occluder 1 corresponds to the open end, which is farther away from an operator, while the proximal end of the occluder 1 corresponds to the closed end, which can be detachably coupled to a push member 62 (e.g., a delivery rod or guidewire) of a delivery device 60 to effectuate delivery, implantation and detachment. In addition, upper end of the occluder 1 corresponds to the distal end, while the lower end thereof corresponds to the proximal end, as viewed in the orientation of Figs. 1 to 4.

In an alternative exemplary embodiment, the occluder 1 further includes a converging member 40 and a graft 50. The converging member 40 is coupled to a proximal end of the mesh body 10. Optionally, the converging member 40 may include a plurality of converging struts 41 and an attachment member 42. The attachment member 42 may be arranged along the axis A, and the plurality of converging struts 41 may surround the attachment member 42, and they are radially connected to the connector 42along the circumferential direction. And, the converging strut 41 is coupled to the mesh body 10.

The attachment member 42 of the converging member 40 is configured for detachable attachment to the push member 62 of the delivery device 60, which may be accomplished by various approaches, including but not limited to, snap, threaded, electrolytic and magnetically attractive attachment. Thus, the occluder 1 may be actuated by manipulating the push member 62. The graft 50 covers an outer circumference of the mesh body 10 to enhance closure of the closed end of the occluder 1 and facilitate sealing apposition of the occluder 1 with the LAA. The graft 50 may further extend to cover each converging strut 41 or a part thereof.

Optionally, the mesh body 10, the cushioning elements 20 and the converging member 40 may each be made of a shape-memory metal, thus possessing a certain degree of self-expandability. Referring to Fig. 6, the occluder 1 may be crimped into the collapsed configuration, where the mesh body 10, the cushioning elements 20 and the converging member 40 are compressed and deformed, whilst the graft 50 creases and folds, leading to a reduced overall outer diameter of the occluder 1, which allows for loading of the occluder 1 into the sheath 63 of the delivery device 60. After that, the occluder 1 may be delivered together with the sheath 63 through blood vessels to a target site for release (into the LAA), and the push member 62 may be operated to push the occluder 1 out of the sheath 63. During this stage, the occluder 1 may expand towards and into the expanded configuration that occludes the cavity of the LAA. It will be understood that apposition of the occluder 1 with a wall of the LAA may be attained, with the occluder 1 being in the fully expanded configuration, or in an intermediate configuration between the collapsed and expanded configurations (where it is not completely expanded due to compression by the LAA wall). However, the present invention is not so limited.

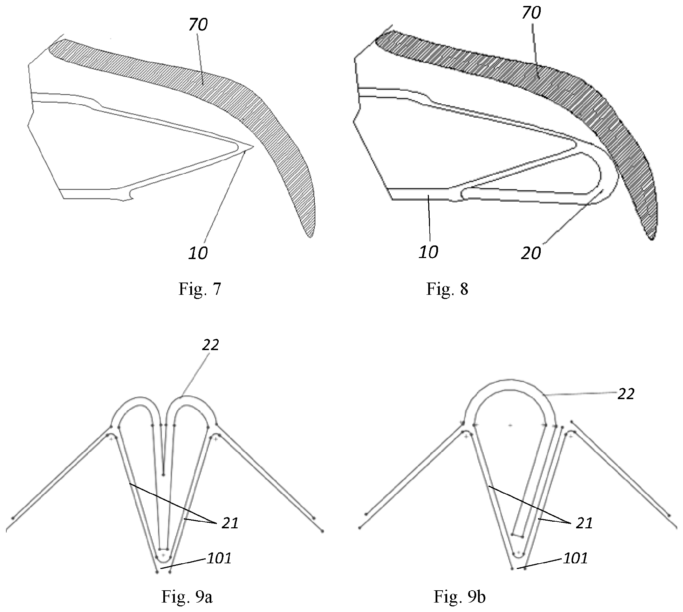

In the expanded configuration of the occluder 1, the plurality of cushioning elements 20 are circumferentially arranged y about the axis A and proximal ends of the cushioning elements 20 is coupled to a distal end of the mesh body 10. Moreover, in this configuration, distal ends of the cushioning elements 20 are free. Preferably, the distal (free) ends of the cushioning elements 20 may be blunt and rounded in shape. For example, these ends may comprise an arcuate shape, elliptic shape or otherwise curved shapes. Referring to Fig. 7, if the cushioning elements 20 were omitted, distal end of the main body 10 often has a relatively sharp portion due to the need of folding, which can easily damage the wall of the LAA 70. In contrast, with the configuration of the cushioning elements 20 which comprise the blunt and rounded distal ends, damage to the wall of the LAA 70 can reduced or eliminate, as shown in Fig. 8.

With continued reference to Figs. 1 to 4, in the expanded configuration of the occluder 1, the cushioning elements 20 may be optionally inwardly inclined towards the axis A along a direction away from the mesh body 10. Here, the direction of the cushioning elements 20 away from the mesh body 10 corresponds to the direction of the cushioning elements 20 towards to distal end. That is, they may be inwardly inclined towards the axis A while extending distally. With this arrangement, the occluder 1 may be overall tapered at the distal end. Such a shape allows both for better apposition of the occluder 1 with the LAA wall when in the expanded configuration and for reduced damage to the LAA wall.

Optionally, when the occluder 1 is in the expanded configuration, each cushioning element 20 may comprise either a planar shape extending perpendicular to its own reference plane 30, or a three-dimensional shape. Optionally each cushioning element 20 may be constructed from a number of struts. In some embodiments, each cushioning element 20 is generally a two-dimensional planar structure, and the plane in which it is located is preferably perpendicular to its own reference plane 30. It will be understood that, in these embodiments, the plane in which the cushioning element 20 is located is not necessarily parallel to the axis A, but may form an angle with the axis A. Preferably, each cushioning element 20 is inclined towards the axis A while extending distally. In some alternative embodiments, each cushioning element 20 is overall a three-dimensional shape, that is, a three-dimensional structure. For example, some struts in each cushioning element 20 may be curved inwardly or outwardly. Compared with a two-dimensional planar shape, the three-dimensional shape may provide larger space for the blunt and rounded shape of the distal end of the cushioning element 20 to allow the blunt and rounded shape portion to have a larger radius of curvature. This can reduce or eliminate damage caused by the cushioning elements 20 to tissue during and after implantation.

Several examples of the occluder 1 are described below with reference to the accompanying drawings.

First of all, referring to Fig. 4, for ease of description and understanding, when the occluder 1 is in the expanded configuration, the mesh body 10 is defined as a three-dimensional mesh structure constructed from a number of interconnected wave struts. At different transverse positions of the mesh body 10 (i.e., different positions perpendicular to the axis A, which can also be understood as different rows), the wave struts of the same type are categorized as one kind of wave strut (including the first wave strut 11, the second wave strut 21, the third wave strut 13, and the fourth wave strut 14, etc., as described below). Joints of struts are referred to nodes (including first nodes 101, second nodes 202 and third nodes 103, as described below).

As shown in Fig. 4, in a first optional example, the mesh body 10 includes a plurality of first wave struts 11 arranged side by side circumferentially about the axis A, and each cushioning element 20 includes two second wave struts 21 and a curved strut 22. The two second wave struts 21 are joined at their one ends to respective two circumferentially adjacent first wave struts 21 and two first nodes 101 are formed, and the other ends of the two second wave struts 21 are joined to each other to form a second node 202. One end of the curved strut 22 is joined to the second node 202, and the other end of the curved strut 22 is joined to the mesh body 10 at an arbitrary location, for example, by snap engagement, sewing, adhesive bonding, welding, or the like. Optionally, the other end of the curved strut 22 may be joined to the mesh body 10 at a certain node. It will be understood that, in some embodiments, the curved strut 22 may be curved inwardly or outwardly with respect to the mesh body 10 in order to be joined to the mesh body 10, in this case, the cushioning element 20 comprises a three-dimensional shape. In some alternative embodiments, the curved strut 22 may be curved laterally within a plane defined by the two second wave struts 21 in order to be joined to the mesh body 10, in this case, the cushioning element 20 comprises a two-dimensional planar sha.

With continued reference to Fig. 4, in one optional example, the other end of the curved strut 22 may be fixed at the first node 101, and the other ends of the curved struts 22 of circumferentially adjacent cushioning elements 20 may be fixed at different first nodes 101. In the exemplary embodiment shown in Fig. 4, the first wave strut 11 is parallel to the axis A, the distal end of the first wave strut 11 is joined to proximal end of the second wave strut 21 and the first node 101 is formed. It will be understood that each cushioning element 20 comprises two second wave struts 21 that are joined to two corresponding first wave struts 11.

Referring to Figs. 9a and 9b, in the other ends of the curved struts 22 of circumferentially adjacent cushioning elements 20 are fixed at different first nodes 101, that is, at each first node 101, there is at most a single curved strut 22 being joined. It will be understood that, when circumferentially adjacent first nodes 101 are spaced at determined intervals, more curved struts 22 joined thereat mean a smaller space being left for each curved strut 22, and the reduced space will reduce a radius of curvature of bending of the distal end of the curved strut 22 (see Fig. 9a). Therefore, the arrangement with at most a single curved strut 22 being joined at each first node 101 (see Fig. 9b) can maximize the radius of curvature of the bending of the distal end of the curved strut 22. It will be understood that a larger radius of curvature can provide for better protection for tissue. As will be appreciated, an infinitely small radius of curvature would correspond to a sharp point capable of causing significant damage. On the contrary, a larger radius of curvature would correspond to a shape that more resembles a sphere and provides increased safety.

Further, each cushioning element 20, when including a single curved strut 22, will have a relatively small footprint after being retracted into the sheath 63, allowing for easier retrieval and delivery. Moreover, in the case of each cushioning element 20 including a single curved strut 22, the cushioning elements 20 and the distal end of the mesh body 10 can be easily folded and flattened, reducing their footprint within the sheath 63. Here, the term "flattened" can be interpreted as transforming the three-dimensional distal end into a two-dimensional form as much as possible. Conversely, if each cushioning element 20 includes multiple curved struts 22, overlapping of these curved struts 22 may occur when the cushioning elements 20 are crimped, leading to lesser degree of flattening, and hence a larger footprint in the sheath 63, compared to the case of a single curved strut 22.

Furthermore, in most cases, when each cushioning element 20 includes a single curved strut 22, the struts in the cushioning elements 20 are allowed to have a smaller surface area, resulting in a reduced total area of the implant, when compared to the case of each cushioning element 20 including multiple curved struts 22. This is advantageous, in particular when the cushioning elements 20 are metal-based. For example, when the cushioning elements 20 are made of a nickel-titanium alloy, a smaller surface area helps reduce the leaching of nickel ions.

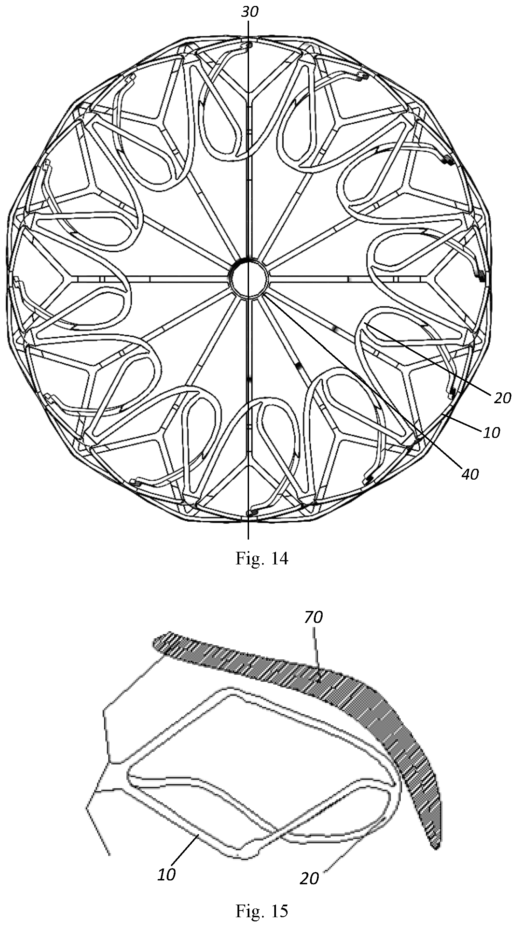

It should be noted that, in some embodiments, the blunt and rounded distal ends of the cushioning elements 20 may be solely provided by bending of distal ends of the curved struts 22, as shown in Figs. 4, 10 and 11. However, in some alternative embodiments, the blunt and rounded distal ends of the cushioning elements 20 may be provided by bending of both the curved struts 22 and the second wave struts 21, as shown in Figs. 12 to 15. The radius of curvature of the blunt and rounded distal ends of the cushioning elements 20 is affected primarily by factors, including a length of the curved struts 22 themselves, the locations where the curved struts 22 are joined to the mesh body 10, and a length of the second wave struts 21. In particular, the main factors that affect the radius of curvature of the blunt and rounded distal ends of the cushioning elements 20 (or radius of curvature of bendings of the distal ends of the curved struts 22) may be as follows:
1) three-dimensional size of overall structure: The number of cushioning elements 20 affects the three-dimensional size of the overall structure. With the overall structure kept unchanged, more cushioning elements 20 mean a smaller space left for each cushioning element 20, and hence a reduced maximum allowable radius of curvature for the cushioning elements 20.
2) length of curved struts: with length of the second wave struts 21, locations of the first and second nodes 101, 202, as well as values of all the other parameters kept unchanged, the greater the length of the curved struts 22 is, the larger the radius of curvature of the bending of the distal end thereof is.
3) locations of joints of the curved struts and the mesh body: with length of the curved struts 22, length of the second wave struts 21 and values of all the other parameters kept unchanged, the more distal the locations of the joints of the curved struts 22 and the mesh body 10 are (i.e., the closer they are to the upper end, as viewed in the orientation of Fig. 4), the greater the radius of curvature of the bending of the distal end of the curved struts 22 is. For example, if the proximal end of the curved strut 22 is connected to the proximal node of the first wave strut 11, then the radius of curvature of the bending of the distal end of the curved strut 22 will be smaller than if it is connected to the node of the distal end of the first wave strut 11 (i.e., the first node 101).
4) length of second wave struts: with length of the curved struts 22, locations of the joints of the curved struts 22 and the mesh body 10 and values of all the other parameters kept unchanged, the shorter the length of the second wave struts 21 is, the greater the radius of curvature of the bending of distal end of the curved strut 22 is.

A person of skill in the art may properly configure the radius of curvature of the blunt and rounded distal ends of the cushioning elements 20 (or the radius of curvature of the bending of the distal end of the curved struts 22), as required by the application, by taking into account the factors discussed above.

The mesh body 10 may further include a plurality of third wave struts 13 each inclined relative to the axis A The circumferentially adjacent third wave struts 13 have opposite inclination directions. The circumferentially adjacent third wave struts 13 may be joined end to end to form a zigzag-shaped ring. Each of the first wave struts 11 may be proximally joined to a distal end of a corresponding the third wave struts 13. Other structural details of the mesh body 10 may be as known in the art and, therefore, need not be described in further detail herein.

Optionally, the distal ends of the cushioning elements 20 may be inwardly inclined. The joint of the second wave strut 21 and the first wave strut 11 is at an angle in the radial direction. Preferably, exterior of the first nodes 101 (on the side away from the axis A) may be rounded to reduce damage that may occur otherwise due to the presence of sharp transitions there.

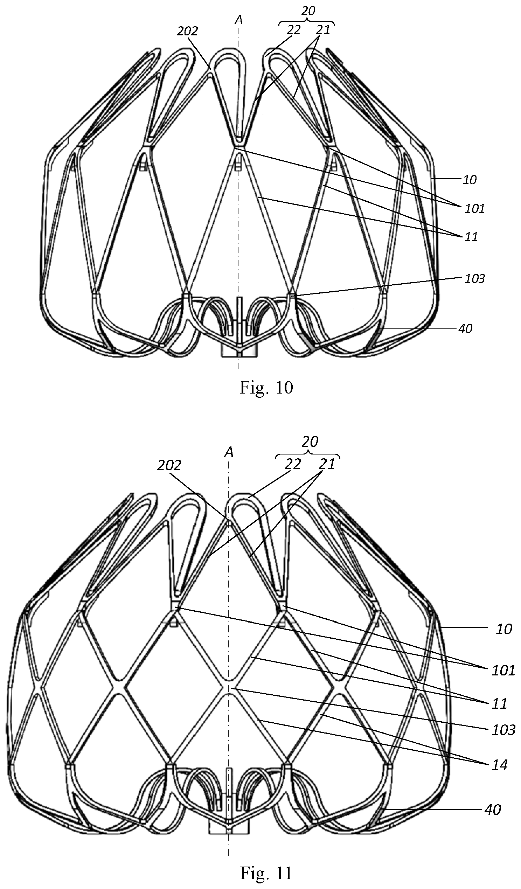

Referring to Figs. 10 and 11, in another optional example, each of the first wave struts 11 is inclined relative to the axis A. The circumferentially adjacent first wave struts 11 have opposite inclination directions, and the plurality of first wave struts 11 are joined sequentially to form a zigzag-shaped ring. The ends of circumferentially adjacent first wave struts 11 towards the cushioning elements 20 (i.e., their distal ends) are joined together and are further joined to the second wave struts 21 to form the first nodes 101. Moreover, the other end of each curved strut 22 is fixed at the first node 101, and the other ends of the curved struts 22 of circumferentially adjacent cushioning elements 20 are fixed at e different first nodes 101.

Unlike the exemplary embodiment of Fig. 4, in the exemplary embodiment shown in Figs. 10 and 11, the first wave strut 11 is inclined relative to the axis A, and adjacent first wave struts 11 are joined end to end to form a zigzag-shaped ring. It will be understood that the joint of each two adjacent first wave struts 11 forms a node (corresponding to an upper apex in the zigzag-shaped ring, as viewed in the orientation of Figs. 10 and 11) towards the distal end, or forms a node (corresponding to a lower apex in the zigzag-shape ring, as viewed in the orientation of Figs. 10 and 11) towards the proximal end. Those nodes towards the distal end correspond to the first nodes 101 and the nodes towards the proximal end correspond to third nodes 103.

Proximal ends of the two second wave struts 21 in each cushioning element 20 are joined at two circumferentially adjacent first nodes 101, with the proximal end of the curved strut 22 in the same cushioning element 20 being fixed at one of the first nodes 101. Moreover, the curved struts 22 in circumferentially adjacent cushioning elements 20 may be fixed at different first nodes 101. This ensures that there is at most a single curved strut 22 being joined at each first node 101.

Optionally, in some embodiments, the mesh body 10 may include only one row of struts. That is, the mesh body 10 may include one row of first wave struts 11, which forms a zigzag-shaped ring. In these embodiments, the first wave struts 11 may be directly joined to the converging member 40 at the third nodes 103, as shown in Fig. 10. In some other embodiments, the mesh body 10 may include two or more rows of struts. As shown in Fig. 11, the mesh body 10 may include a row of first wave struts 11 which forms a zigzag-shaped ring, and a row of fourth wave struts 14 which forms another zigzag-shaped ring. The structure of the zigzag-shaped ring formed by the fourth wave struts 14 may be similar to the structure of the zigzag-shaped ring formed by the first wave struts 11, and is coupled to a proximal end of the zigzag-shaped ring formed by the first wave struts 11. In these embodiments, the first wave struts 11 may be joined to the fourth wave struts 14 at the third nodes 103 and thereby connected to the converging member 40 through the fourth wave struts 14.

Referring to Figs. 12 to 15, in a third optional example, each of the first wave struts 11 is inclined relative to the axis A in a direction opposite to a direction in which any circumferentially adjacent one of the first wave struts 11 is inclined relative to the axis A, and circumferentially adjacent first wave struts 11 are joined together to form a zigzag-shaped ring. The ends of circumferentially adjacent first wave struts 11 proximal to the cushioning elements 20 (i.e., their distal ends) are joined together and are further joined to the second wave struts 21, and the first nodes 101 are formed. Moreover, the ends of circumferentially adjacent first wave struts 11 away from the cushioning elements 20 (i.e., their proximal ends) are joined together and the third nodes 103 are formed. The other end of each curved strut 22 is fixed at the third node 103, and the other ends of the curved struts 22 of circumferentially adjacent cushioning elements 20 are fixed at different third nodes 103.

Unlike the exemplary embodiment of Figs. 10 and 11, in the exemplary embodiment shown in Figs. 12 to 15, the other ends of the curved struts 22 are fixed at the third nodes 103, instead of at the first nodes 101. With this arrangement, the curved struts 22 are allowed to have an increased length, and hence a larger radius of curvature of their blunt and rounded distal ends. Preferably, one end of each curved strut 22 is joined at a second node 202, and the other end thereof is inwardly curved so as to extend inside the mesh body 10 to a third node 103. Of course, in some alternative embodiments, each curved strut 22 may also be curved outwardly from the mesh body 10 so as to extend to a third node 103.

Further, the other end of the curved strut of each cushioning element is joined to a third node 103 formed by two first wave struts 11 that are joined to the two second wave struts 21 of the same cushioning element 20. In other words, the other end of the curved strut of each cushioning element is joined at a third node 103 corresponding to the same cushioning element 20. Thus, it will be understood that since each cushioning element 20 comprises two second wave struts 21, the two second wave struts 21 are required to be joined to the distal ends of two first wave struts 11, while the proximal ends of these two first wave struts 11 are joined to form the third nodes 103, the corresponding cushioning element 20 corresponds to the third node 103 at the circumferential position (i.e., they can be considered as belonging to the same column). Therefore, the proximal end of the curved strut 22 of each cushioning element 20 may be configured to be fixed at a third node 103 corresponding to the same cushioning element 20 in terms of circumferential position can reduce the risk of circumferential entanglement or twisting of the curved strut 22.

Optionally, referring to Fig. 16, in some alternative embodiments, the other end of the curved strut 22 of each cushioning element 20 may be joined at a third node 103 corresponding to a circumferentially adjacent cushioning element 20. Unlike the exemplary embodiment of Fig. 13, in the embodiment shown in Fig. 16, the curved strut 22 of each cushioning element 20 circumferentially extends toward an adjacent cushioning element 20 and is joined at a third node 103 corresponding to the adjacent cushioning element 20 in terms of circumferential position. This embodiment allows for an even larger radius of curvature of the curved struts 22.

Optionally, at least one of the second wave struts 21 of each cushioning element 20 may have a curved section 211 at its end away from the first nodes 101. In this case, each second wave strut 21 with such a curved section 211 at its distal end is no longer a straight strut. Preferably, each such second wave strut 21 is curved at its distal end towards its joint with the curved strut 22 so as to form, together with the curved strut 22, a larger blunt and rounded shape with an even greater radius of curvature.

It will be understood that Figs. 4 and 10 to 16 show merely several non-limiting exemplary embodiments of the cushioning elements 20 and mesh body 10, and in some alternative embodiments, the mesh body 10 may include more rows of struts, and/or there may be another number of cushioning elements 20 than as shown in Figs. 4 and 10 to 16. A person of skill in the art can select any appropriate configuration, as required by the application.

With continued reference to Fig. 6, embodiments disclosed herein also provide an occlusion system including an occluder 1, as discussed above, and a delivery device 60 configured for loading therein of the occluder 1 in the collapsed configuration and for release of the occluder 1 therefrom.

In an alternative exemplary embodiment, the delivery device 60 includes an attachment member, a push member 62 and a sheath 63. The push member 62 may be, for example, a delivery rod or guidewire, and the attachment member 61 may be affixed at a distal end of the push member 62. Both the push member 62 and the attachment member 61 may be movably inserted in the sheath 63. The attachment member 61 is configured to be detachably coupled to the converging member 40 of the occluder 1. Once the attachment member 61 is coupled to the converging member 40, the sheath 63 may be distally displaced to load the occluder 1 into the sheath 63. At this point, the occluder 1 is in the collapsed configuration.

After the distal end of the sheath 63 is advanced through blood vessels to LAA, a proximal end of the push member 62, which remains outside the patient's body, may be manipulated to push the occluder 1 out of the sheath 63 through its distal end. After the occluder 1 is so released, the attachment member 61 may be detached from the converging member 40, with the occluder 1 being retained in the LAA. The sheath 63 may be then withdrawn, ending the implantation process.

In summary, the present invention provides an occluder and an occlusion system. The occluder includes a mesh body and a plurality of cushioning elements, and has open and closed ends long an axis. The occluder comprises a collapsed configuration and an expanded configuration. When the occluder is in the expanded configuration, the mesh body comprises a ring shape around the axis, and the plurality of cushioning elements are arranged at the open end circumferentially about the axis. A perpendicular bisector plane of the cushioning element passing through the axis, defines a reference plane. Each cushioning element is asymmetrical about its reference plane. With this arrangement, the asymmetry arranged of each cushioning element about its reference plane can effectively reduce entanglement or interdigitating of the cushioning elements, thereby enhancing the stability of the occluder without compromising its performance during use. In addition, the presence of the cushioning elements helps reduce damage to tissue, contributing to increased safety thereof.

It should be noted that the foregoing embodiments may be combined in any suitable combination. The description presented above is merely that of some preferred embodiments of the present invention and is not intended to limit the scope thereof in any sense. Any and all changes and modifications made by those of ordinary skill in the art based on the above teachings fall within the scope of the invention.

## Claims

1. An occluder, comprising a mesh body and a plurality of cushioning elements,
wherein the occluder comprises an open end and a closed end along an axis, and comprises a collapsed configuration and an expanded configuration, wherein when the occluder is in the expanded configuration, the mesh body comprises a ring shape about the axis, and wherein the plurality of cushioning elements are circumferentially arranged at the open end about the axis,
wherein a perpendicular bisector plane of the cushioning element passing through the axis defines a reference plane, and wherein the cushioning element is asymmetrical about the reference plane.

2. The occluder according to claim 1, wherein when the occluder is in the expanded configuration, each cushioning element is inwardly inclined towards the axis along a direction away from the mesh body.

3. The occluder according to claim 1, wherein when the occluder is in the expanded configuration, each cushioning element comprises a planar shape perpendicular to the reference plane, or a comprises a three-dimensional shape.

4. The occluder according to claim 1, wherein the mesh body comprises a plurality of first wave struts circumferentially arranged about the axis,
wherein each cushioning element comprises two second wave struts and a curved strut, wherein the first ends of the two second wave struts joined to two circumferentially adjacent first wave struts, thereby forming two first nodes, and wherein second ends of the two second wave struts are joined to each other to form a second node,
wherein a first end of the curved strut is joined to the second node, and wherein a second end of the curved strut is joined to the mesh body.

5. The occluder according to claim 4, wherein the second end of the curved strut is affixed at the first node, and wherein the second ends of the curved struts of circumferentially adjacent cushioning elements are affixed at different first nodes.

6. The occluder according to claim 5, wherein the first wave strut is inclined relative to the axis, wherein circumferentially adjacent first wave struts comprise opposite inclination directions, wherein the plurality of first wave struts are joined sequentially to form a zigzag-shaped ring, and wherein ends of circumferentially adjacent first wave struts towards the cushioning elements are joined together and are further joined to the second wave strut to from the first node.

7. The occluder according to claim 4, wherein the first wave strut is inclined relative to the axis, wherein circumferentially adjacent first wave struts comprise opposite inclination directions, wherein the plurality of first wave struts are joined sequentially to form a zigzag-shaped ring; ends of circumferentially adjacent first wave struts towards the cushioning elements are joined together and are further joined to the second wave strut to form the first node, and wherein ends of circumferentially adjacent first wave struts away from the cushioning element are joined together to form a third node; and
wherein the second end of the curved strut is affixed at the third node, and wherein the second ends of the curved struts of circumferentially adjacent cushioning elements are affixed at different third nodes.

8. The occluder according to claim 7, wherein the second end of the curved strut of the cushioning element is joined to the third node formed by the first wave struts that are joined to the two second wave struts of the same cushioning element.

9. The occluder according to claim 4, wherein, in each cushioning element, one end of at least one of the second wave struts away from the first node comprises a curved section, and/or wherein the curved strut is curved inwardly or outwardly with respect to the mesh body.

10. An occlusion system, comprising an occluder as defined in any one of claims 1 to 9 and a delivery device, wherein the delivery device is configured to load the occluder in the collapsed configuration and to release the occluder.
